# FASCICULE DE BREVET EUROPEEN

(11) **EP 0 485 491 B1**
(45) Date de publication et mention de la délivrance du brevet: **01.03.1995**
(21) Numéro de dépôt: 90912527.0
(22) Date de dépôt: 20.07.1990
(51) Int. Cl.: C07K 14/78, A61K 38/39

(54) **MEMBRANE BIOLOGIQUE ARTIFICIELLE**
KÜNSTLICHE BIOLOGISCHE MEMBRAN
ARTIFICIAL BIOLOGICAL MEMBRANE

(30) Priorité: 20.07.1989 FR 8909798
(43) Date de publication de la demande: 20.05.1992
(73) Titulaire: INSTITUT NATIONAL DE LA SANTE ET DE LA RECHERCHE MEDICALE (INSERM), 75654 Paris Cédex 13 (FR)
(72) Inventeur: RABAUD, Michel, F-33400 Talence (FR); LEFEBVRE, Françoise, F-33160 S.-Médard-en-Jalles (FR)
(74) Mandataire: Peaucelle, Chantal
(86) Numéro de dépôt international: FR9000554
(87) Numéro de publication internationale: WO9101393

(56) Documents cités:
- EP-A- 0 128 706
- WO-A-86/05097
- US-A- 4 776 853

## Description

L'invention a pour objet des compositions de matériaux protéiques possédant notamment des propriétés d'élasticité et de robustesse. Elle vise également leur préparation et leurs applications biologiques, biochimiques et pharmacologiques, en particulier pour l'élaboration de membranes biologiques artificielles.

L'expression "membrane biologique artificielle" recouvre, dans son acception la plus générale, des membranes obtenues à partir de produits d'origine biologique et utilisées dans des domaines très divers, tels que la chirurgie, la médecine, la pharmacie mais aussi l'industrie chimique, textile ou du papier.

Les propriétés requises pour ces applications sont esentiellement des propriétés d'élasticité, de bonne tenue mécanique, de résistance chimique et de compatibilité avec des milieux divers.

Comme constituant de telles membranes, on a déjà proposé d'utiliser de l'élastine en mettant à profit ses propriétés d'élasticité et son caractère à la fois hydrophile et hydrophobe.

Ainsi, dans la demande FR n_{°} 8503057 du 01.03.85 au nom de l'INSERM, on a rapporté un produit à base d'élastine et de fibrine soluble (à savoir de monomères de fibrine) particulièrement approprié pour l'élaboration de biomatériaux et matériaux supports, tels que tissus artificiels ou supports de cultures cellulaires.

La recherche de matériaux plus spécialement adaptés pour effectuer des greffes sur des vaisseaux de petit calibre a conduit l'inventeur de la présente demande à étudier le comportement de différents produits biologiques vis-à-vis d'une trame de matériau polymère servant de support.

Les travaux effectués ont montré gu'en faisant réagir de l'élastine ou des dérivés d'élastine, dans certaines conditions, avec une autre protéine du tissu conjonctif, on obtient un matériau protéique adhérant fortement au matériau support utilisé, possédant les propriétés de protéines structurelles et répondant ainsi, de manière particulièrement satisfaisante, aux exigences requises envers les membranes biologiques.

L'invention a donc pour but de fournir de nouvelles compositions de matériaux protéiques dotées de propriétés importantes d'élasticité, de robustesse et, le cas échéant, d'adhésivité et conservant leurs propriétés avec divers additifs.

Elle vise également à fournir un procédé permettant d'obtenir aisément ces compositions.

L'invention a également pour but de fournir des membranes biologiques utiles plus spécialement en tant que tissus conjonctifs artificiels et comme matériaux supports pour des cultures cellulaires.

Les compositions de matériaux protéiques de l'invention sont essentiellement telles qu'obtenues par réaction d'une solution aqueuse concentrée de collagène de type 1 et/ou III et d'élastine ou de peptides d'élastine de poids moléculaire (PM) supérieur à environ 10.000, solubles dans l'eau, tels qu'obtenus par solubilisation de l'élastine.

Dans la suite de la description, ces peptides d'élastine dont il est question ci-desus seront appelés "peptides solubilisés d'élastine" et désignés par l'abbréviation PSE.

Une telle composition présente des qualités d'élasticité et de tenue mécanique permettant de la comparer aux protéines structurelles. Elle possède également des propriétés d'adhésivité élevées particulièrement précieuses pour l'utilisation dans les applications biologiques envisagées.

Une composition de matériau protéique préférée est essentiellement telle qu'obtenue par réaction d'élastine et de collagène de type 1 et de type III. La proportion de collagène de type III par rapport à celle de collagène de type est avantageusement supérieure à 50% en poids, en particulier d'au moins 70% en poids (poids sec). On notera que le collagène de type III tel que commercialisé, renferme de telles quantités de collagène de type I.

L'élastine est une élastine d'origine humaine ou animale, native ou mature, ou le cas échéant, leurs dérivés. Il s'agit généralement d'élastine extraite de tissus animaux, en particulier de ligament nucal de boeuf, ou d'aorte de porc, de bovidé ou humaine.

Une autre composition préférée de l'invention est essentiellement telle qu'obtenue par réaction de peptides solubilisés d'élastine et de collagène de type III.

Le collagène utilisé dans ces compositions, selon une disposition de l'invention, est du collagène extrait de placenta humain.

Ces compositions, contrairement à l'élastine, ne donnent pas de réaction avec les monomères de fibrine.

Dans une disposition préférée de l'invention, le rapport élastine ou PSE/collagène (p/p, par rapport au poids sec) est de 10 à 1 environ, de préférence de l'ordre de 2,5.

En variante, les compositions de l'invention comprennent un matériau composite dans lequel les PSE et le collagène sont fixés à un matériau de substitution de l'os.

Le matériau de substitution de l'os est avantageusement choisi parmi les matériaux biocompatibles et dotés de propriétés d'ostéoconduction (c'est-à-dire capables d'être recolonisés par les structures organiques ou vivantes susceptibles de fabriquer l'os, grâce à leurs propriétés de surface et leur porosité) et d'ostéinduction (c'est-à-dire capables de créer un environnement favorable au déclenchement de l'ostéogénèse).

De tels matériaux sont notamment à base de Ca⁺⁺, de phosphate tricalcique ou d'apatites. Un matériau particulièrement approprié est constitué par l'hydroxyapatite.

L'invention vise ainsi des compositions telles qu'obtenues par réaction d'hydroxyapatite avec des PSE et du collagène de type 1 et/ou III.

Le procédé de préparation des compositions de matériaux protéiques définies ci-dessus est caractérisé en ce qu'il comprend:
- la mise en contact, avec une solution aqueuse concentrée de collagène de type 1 et/ou III, dans des conditions permettant la formation d'un gel, d'élastine ou de peptides d'élastine de PM au moins égal à environ 10.000, ces peptides étant solubles dans un mélange 50-50 d'alcool tertiobutylique-potasse 1 M dans de l'eau,
- la récupération du gel formé et son traitement ultérieur, si souhaité.

La concentration en collagène de la solution aqueuse est d'environ au moins 3 mg/ml, notamment de l'ordre de 3 à 10 mg/ml.

Selon un mode préféré de réalisation de l'invention, l'étape de mise en contact est réalisée dans des conditions physiologiques, à savoir à 37 ° C environ, à un pH de l'ordre de 7 à 7,5.

L'élastine ou les PSE sont respectivement en suspension ou dissous dans un tampon de pH de 7 à 7,5 environ, de préférence de pH 7,4, dont les éléments constitutifs favorisent ou tout au moins n'inhibent pas la réaction recherchée. Des tampons renfermant par exemple, des cations, Na⁺, Ca⁺⁺ et/ou Mg⁺⁺, des anions PO₄= et/ou C1- apparaissent avantageux à cet égard.

Un tampon particulièrement approprié, qui renferme l'ensemble des ions ci-dessus, est constitué par le tampon PB qui répond à la composition molaire suivante : 1mM P0₄, 150mM NaCI, 2mM Ca⁺⁺, 1mM Mg⁺⁺.

Des gels particulièrement résistants à l'écrasement sous le doigt sont obtenus à partir d'élastine ou de PSE de tampon et de collagène utilisés dans un rapport d'environ 2,5.

Ce rapport peut être modifié selon l'application de la composition.

L'élastine est généralement obtenue à partir de tissus animaux par dissolution et élimination des protéines d'accompagnement.

Comme tissus, on utilise de préférence le ligament nucal de boeuf ou l'aorte de porc ou de bovidé.

Les peptides d'élastine résultent par exemple de l'hydrolyse de l'élastine par traitement alcalin notamment avec de la potasse 1 M, traitement acide, par exemple avec de l'acide oxalique ou traitement enzymatique avec de l'élastase.

Le collagène provient plus spécialement de placenta humain.

Pour disposer de membranes utilisables dans les applications envisagées ci-après, on ajoute successivement aux PSE des additifs permettant d'améliorer les qualités du gel et de lui conférer des propriétés utiles dans les applications envisagées, puis le collagène.

Parmi les additifs avantageux utilisés seuls ou en combinaison, on citera la fibronectine et la laminine, qui favorisent les qualités cohésives du gel, le collagène de type IV qui améliore les qualités adhésives, des hormones de croissance, des éléments nutritifs, des vitamines, des protéoglycanes tels que l'héparane et le dermatane-sulfate, des enzymes, des antiseptiques, des catalyseurs ou des oxydo-réducteurs. Ces additifs conservent la structure des matériaux protéiques de l'invention.

De nombreux autres additifs, agents thérapeutiques, antibactériens, sont utilisables et seront aisément choisis par l'homme de l'art en fonction des propriétés recherchées, et mis en oeuvre selon les doses appropriées compte tenu des applications envisagées.

Le mélange formé est ensuite incubé à une température de l'ordre de 37 ° C jusqu'à la formation du gel. Une dispersion satisfaisante des différents éléments du mélange est assurée par une mélangeur par exemple de type VORTEX^{R}.

Lorsqu'on souhaite imprimer une forme et une dimension donnée à la composition, on réalise la réaction dans un moule approprié, en mettant à profit le caractère modulable des compositions de l'invention. Il est ainsi possible d'obtenir la composition qui sera utilisée comme biomatériau sous forme de tubulures, filaments ou de bandes.

La composition est démoulée et, le cas échéant, séchée pour éliminer l'excès d'eau.

La conservation est effectuée à l'état humide en présence d'un antiseptique ou à l'état lyophylisé. Dans ce cas, le produit est rehydraté par mise en contact avec de l'eau physiologique stérile avant utilisation.

Pour l'élaboration de compositions formées de matériaux composites, on fait avantageusement réagir le matériau de substitution de l'os avec des PSE et ensuite avec du collagène. Les conditions de température et de pH sont en particulier telles qu'indiquées ci-dessus.

Comme déjà rapporté, les compositions de l'invention présentent de remarquables propriétés d'élasticité, de robustesse ainsi qu'une grande étanchéité, des propriétés de biocompatibilité, et le cas échéant d'adhésivité.

Ces propriétés sont particulièrement avantageuses pour l'élaboration de membranes biologiques artificielles.

L'invention vise donc également des membranes caractérisées en ce qu'elles comprennent une composition de matériaux protéiques telle que définie ci-dessus.

Ces membranes présentent un intérêt tout particulier en tant que tissus conjonctifs artificiels.

Par leur constitution, elles sont très proches de la lamina densa des membranes basales, et plus spécialement, lorsqu'elles renferment du collagène type III, du sousendothélium artériel.

Les membranes de l'invention sont utilisables notamment comme pièces d'obturation, de soutien et de renforcement.

Elles peuvent constituer des pièces de soutien temporaire permettant d'initier puis de guider le processus de réparation tissulaire.

Elles sont appliquées à l'aide de colles biocompatibles ou fixées par sutures. En variante, leurs propriétés d'adhésivité élevée permet leur fixation à l'endroit souhaité sans recours à d'autres moyens.

Selon un aspect de grand intérêt de l'invention, ces membranes adhèrent fortement aux matériaux supports polymères habituellement utilisés comme trames dans la préparation de biomatériaux.

On constate en outre qu'elles favorisent la cicatrisation.

On notera que, de manière avantageuse, ces membranes n'induisent pas de phénomène de coagulation étant dépourvues de fibrine et ne se fixant pas à la fibrine.

Dans le traitement des brûlés, elles permettent de compenser certaines pertes de tissus.

Elles présentent aussi un grand intérêt pour fabriquer des vaisseaux artificiels de faible calibre.

D'une manière générale, les membranes de l'invention sont utilisables dans tous les domaines de la chirurgie notamment digestive, vasculaire, urinaire, génitale et obstétricale, maxilo-faciale et plastique, dermatologique, foetale et néonatale ainsi que vétérinaire.

Le choix d'un ou plusieurs additifs particuliers permet de disposer aisément des membranes appropriées pour une application donnée.

Ainsi, en considérant comme additifs par exemple les protéoglycanes, il est possible de préparer des membranes utilisables comme sous-endothélium artériel en utilisant de l'héparane sulfate, ou encore des membranes substituts de cartilage avec des chondroltines sulfates. De même, pour reconstituer un interface épiderme-derme, il est spécialement avantageux d'ajouter aux compositions de l'invention le kératane-sulfate.

L'invention fournit ainsi à l'homme de l'art les moyens de réaliser une membrane adaptée à ses besoins.

Les membranes de l'invention constituent également des matériaux supports particulièrement utiles pour les cultures cellulaires.

Des cultures cellulaires variées peuvent être développées, en opérant dans les conditions habituelles, à l'aide de ces supports. On citera notamment les cultures de cellules musculaires lisses, de fibroblastes, de kératinocytes, de cellules épithéliales et de cellules endothéliales. Les rendements obtenus sont élevés.

Ces membranes jouent également le rôle de socle dermique lorsqu'on ensemence des cellules épithéliales en vue de reconstituer un morceau d'épiderme. Ce dernier est ensuite transplantable sur l'organisme d'un receveur.

Les membranes élaborées à partir des matériaux composites évoqués ci-dessus, plus particulièrement ceux renfermant de l'hydroxyapatite avec des PSE et du collagène sont utilisables comme supports de culture d'ostéoblastes.

Les membranes à base de matériaux composites se présentent sous forme de gels. Les culots de centrifugation sont utilisables dans des études biochimiques.

L'invention vise également des kits renfermant les éléments nécessaires pour effectuer une réparation tissulaire ou une culture cellulaire.

On rapporte dans les exemples qui suivent, donnés à titre illustratif, d'autres caractéristiques et avantages de l'invention.

Les figures auxquelles il est fait référence dans ces exemples représentent
- les figures 1 a et 1 b, des photos prises au microscope électronique à balayage, à différents grossissements, de matériaux protéiques de l'invention,
- la figure 2, la variation de la quantité en mg de produit sec en fonction de la quantité de collagène ajoutée en mg (essai avec 40 mg de PSE), et
- la figure 3, la radioactivité, en cpm/mg du culot de centrifugation lavé, correspondant à une composition de l'invention, en fonction de la quantité de collagène ajoutée, et du temps de réaction.

L'élastine utilisée dans ces exemples est de l'élastine commercialisée par SIGMA provenant de ligament nucal de boeuf.

Le collagène de type 1 + III ou le collagène de type III et la fibronectine sont des produits commercialisés par l'Institut Mérieux, obtenus à partir de placenta humain. Le collagène de type 1 + III renferme 73% de type III et 27% de type I.

### EXEMPLE 1 : PREPARATION DE GEL ELASTINE-COLLAGENE

A une suspension d'élastine broyée et tamisée de 40 mg dans 1 ml de tampon PB de pH 7,4 (1mM P0₄, 150mM NaCI, 2mM Ca⁺⁺, 1mM Mg⁺⁺), on ajoute 1 ml de gel de collagène type I + III, soit à 3 mg/ml, soit à 6 mg/ml. On homogénéise au VORTEX^{R} pendant 5 sec, on verse ensuite le mélange dans un moule à 37 ° C sans agiter pendant 15 min., 1, 2, 12 heures. On récupère le produit, on le lave et on le sèche.

En variante, au lieu de verser le mélange dans un moule, on effectue l'incubation dans un récipient puis on sépare le produit formé par centrifugation (10 min/3000t/mn) Le produit récupéré se présente sous forme d'une seule phase homogène.

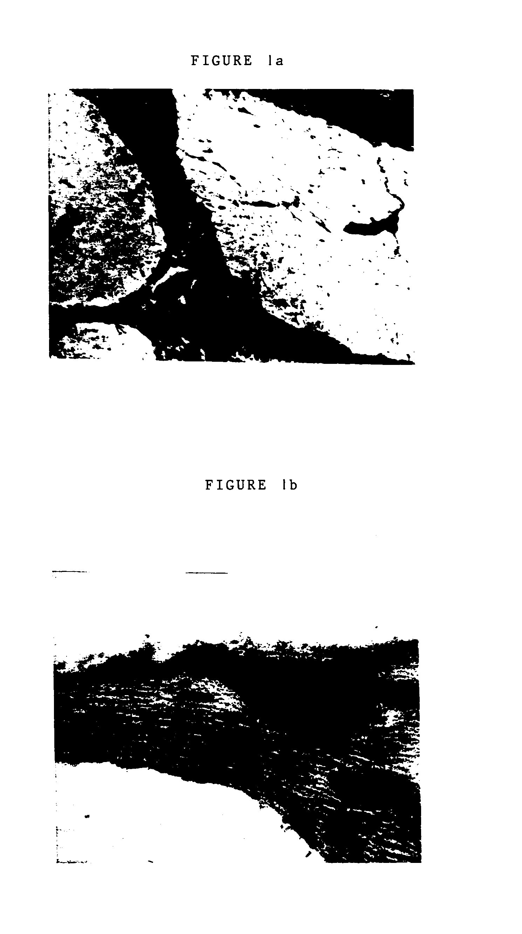

Une étude des structures a été entreprise à l'aide de la microscopie électronique à balayage. Cette technique permet d'observer - en analyse immédiate - une association très étroite entre l'élastine et le collagène (voir figure 1a (grossissement de 5600) et figure 1 b (grossissement 33600)).

### EXEMPLE 2 : PREPARATION DE GEL PEPTIDES SOLUBILISES D'ELASTINE-COLLAGENE

### ^{*} PREPARATION DES PEPTIDES SOLUBLISES D'ELASTINE

On utilise 1 g d'élastine. Après broyage et tamisage, l'élastine est mise en suspension dans 50 ml d'alcool tertiobutylique. On ajoute 50 ml de potasse 1 M dans l'eau, sous agitation suffisante pour maintenir une émulsion. A 25 ° C, on obtient une dissolution complète de l'élastine au bout de 48 heures.

On ajoute alors 50 ml d'eau et on neutralise à pH 7 par addition d'acide acétique.

Par dialyse durant environ 14 heures contre l'eau courante, on élimine l'alcool tertiobutylique, les sels et les peptides de poids moléculaire inférieur ou égal à 10.000 environ.

On effectue ensuite une dialyse contre l'eau permutée (2 bains de deux heures environ).

La préparation est congelée à -40 ° C et lyophylisée aux fins de conservation. Le rendement est de 65 à 85%.

### ETUDE DE LA STABILITE DU GEL OBTENU

50 mg de peptides issus de la solubilisation de l'élastine (marqués avec de l'iode¹²⁵) sont solubilisés dans 2 ml de PB. On ajoute 2 ml de solution de collagène III dans l'eau (10 mg/ml). On homogénéise au VORTEX^{R} (5 sec.). On place au bain- marie à 37 ° C pendant 5 heures. On centrifuge 10 min. à 6.000t/min. Le culot est lavé par 5 ml d'eau pendant 15 min., ensuite par 5 ml d'eau pendant 1 heure, puis par 5 ml d'eau jusqu'au lendemain. Après chaque lavage, on mesure la radio-activité du culot.

Le produit est alors abandonné 7 jours et une autre série de lavage à l'eau est effectuée. Le produit lavé est placé dans l'eau à 25 ° C pendant 18 jours sans changer d'aspect. Après centrifugation, le produit est séché à l'air (25-29 ° C) durant deux jours.

On obtient un matériau compact - alors qu'au cours des lavages, il avait été assez dilacéré pour des raisons d'efficacité de l'opération - et très résistant.A ce moment-là, repris dans l'eau, il fournit une matrice plus plastique qui, en présence d'acide acétique 1/2 (pH ≈ 1), n'est pas solubilisée mais donne à terme un gel assez semblable au premier.

### ^{*} REACTION AVEC LE COLLAGENE DES PSE

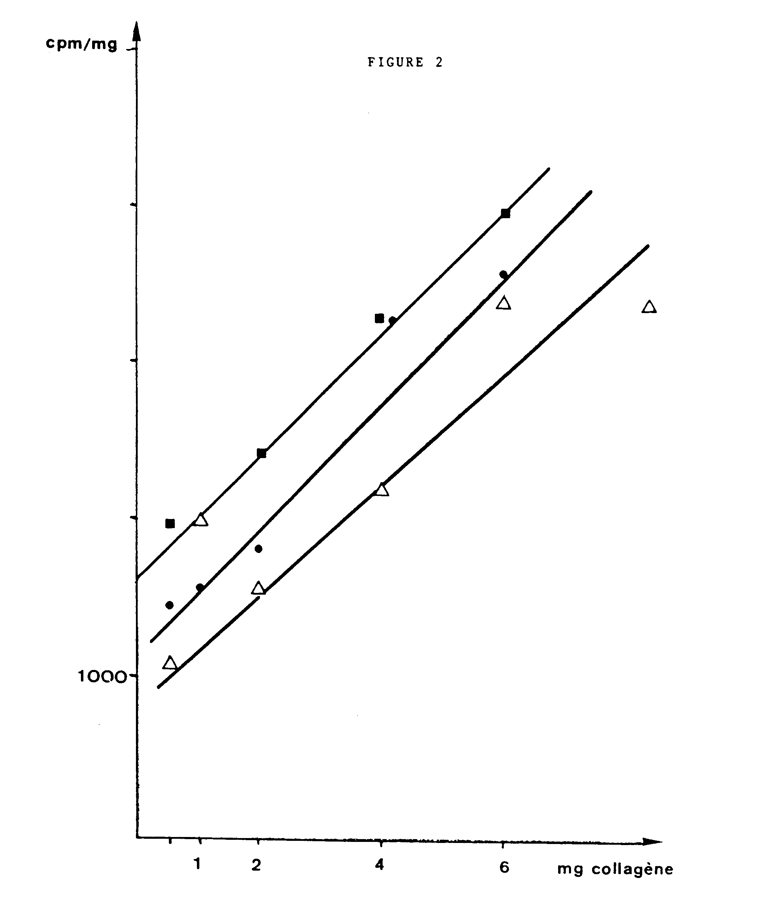

1ère expérimentation : avec PSE, non marqués à l'iode¹²⁵, on met en contact puis homogénéise (5 sec., VORTEX^{R}), des doses croissantes de PSE - de O, 5, 10, 20, 30, 40, 60 et 100 mg dans 1 ml PB 7,4 à 37 C - avec 1 ml du gel de collagène 1 + III à 3mg/ml.
   On observe que les gels se forment d'autant plus rapidement et se présentent sous une forme d'autant plus compacte que la quantité de PSE est élevée.
   La centrifugation, 10 min à 5000 t/min, au bout d'une heure, permet la séparation des gels : de 5 à 20 mg PSE, ils sont mous ; de 30 à 100 mg PSE, beaucoup plus fermes. Les plus résistants à l'écrasement sous les doigts sont les gels obtenus avec 30 et 40 mg de PSE. Les gels ont été amenés à sec, à l'air, et pesés. On observe une certaine proportionalité du poids obtenu en fonction du poids de PSE.
2ème expérimentation : On opère comme indiqué ci-dessus, en utilisant des doses de 10, 20, 30, 40, 60 et 80 mg de PSE dans 1 ml de PB, pH 7,4 et 1 ml de collagène 1 + III.
   Avec 40 mg de PSE, on utilise 1, 2 et 3 ml de solution de collagène.
3ème expérimentation : On opère comme indiqué ci-dessus mais avec une quantité fixe de PSE (40 mg/ml) et des doses croissantes de collagène 1 + III à 10 mg/ml, soit 2, 4, 6, 8, 10, 12, 14 et 20 mg.
   Le gel se prend en masse rapidement à partir de 6 mg, les tubes à 14 et 20 mg sont beaucoup plus opaques. Pour les décanter, on ajoute H₂0/EtOH avant la centrifugation. Tous les gels obtenus sont lavés, et mis à sécher sur papier filtre. On observe une assez bonne proportionalité entre la quantité de produit obtenu après séchage du gel et la quantité de collagène (voir figure 2).
4ème expérimentation : Pour étudier des gels PSE-collagène, on utilise des PSE marqués à l'iode ¹²⁵ et on détermine les évènements produits par la mesure de la radio-activité retenue sur le précipité.

Une solution de PSE-¹²⁵ est préparée à 5 mg/ml et à 4.600 cpm/mg à laquelle on ajoute les doses croissantes de collagène : 0.5, 1, 2, 4 et 6 mg précédé de la quantité suffisante de tampon PB 7,4, pour 2 ml volume final. Le gel se forme bien.

On centrifuge 30 à 40 min. à 5500 t/min. puis on mesure la radio-activité du surnageant, du culot brut, puis du culot lavé 2 fois avec H₂0.

Cette expérience est réalisée sur des temps variables : 1, 2 et 4 heures.

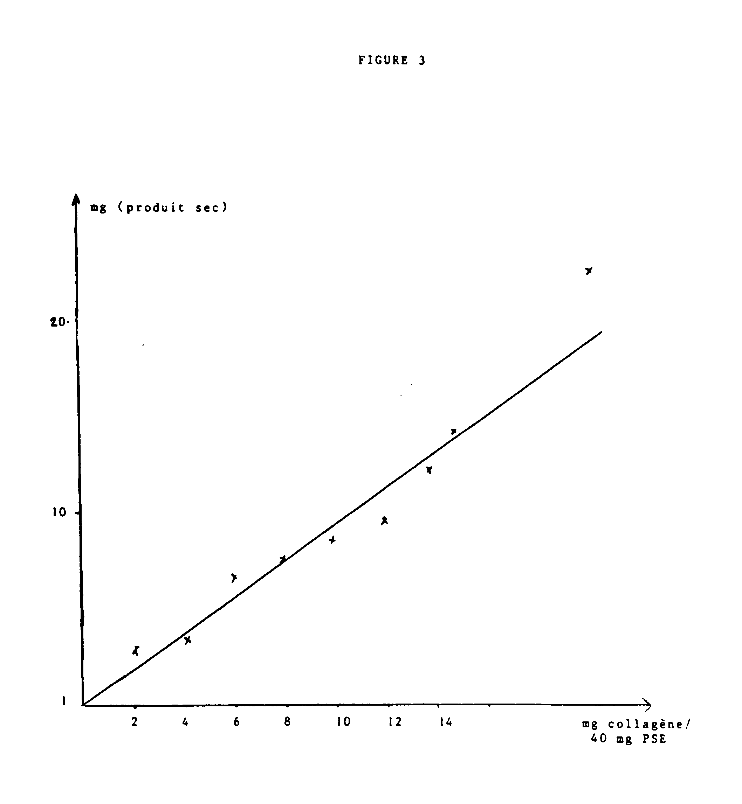

Les résultats obtenus sont rapportés sur la figure 3 qui donne la mesure de la radio-activité (cpm/mg) sur le culot lavé en fonction de la quantité pondérale de collagène, et ce en fonction du temps de l'expérience.

La courbe avec les symboles ■ correspond à une durée d'expérience de 1 heure, celle avec les symboles O, à une durée de 2 heures et celle avec les symboles à une durée de 4 heures.

### EXEMPLE 3 : PREPARATION DE GELS COMPORTANT DE LA FIBRONECTINE, DE LA LAMININE ET DU COLLAGENE DE TYPE IV

On utilise de la fibronectine marquée en solution dans un tampon PO₄ 0,1 M, NaCI 0.15 pH 7,2, à 1,2 mg/ml et 8,10⁶ cmp/mg.

On prépare des PSE à 10 mg/ml PB 7.4. A 1 ml de cette solution, on ajoute 10, 20, 50, 100 et 200 µl de la solution de fibronectine-l¹²⁵. On incube 30 min à 37 ° C et ajoute 0,4 ml de collagène 1 + III et la qsp de PB 7,4 pour 2 ml de volume final. On homogénise au VORTEX^{R} (5sec.) et conserve sans agitation 4 heures à 37 _{°} C. La centrifugation à 5500 t/min. pendant 40 min. permet de séparer culots et surnageants.

On constate que l'addition de fibronectine ac- cèlère la formation du gel : il se forme instantanément pour 200 µl de fibronectine. Des lavages répétés, d'abord courts et dans l'eau, puis 14 heures environ dans l'eau à 20 ° C, ne diminuent que de 17% en moyenne la radio-activité retenue dans le gel et portée par la fibronectine, ce qui démontre l'importance de l'affinité de la fibronectine vis-à-vis des constituants du gel.

On utilise de la laminine (Sigma) et du collagène de type IV (Mérieux) marqués en solution dans le tampon PO₄ 0,1 M utilisé dans l'essai avec la fibronectine - laminine 500.000 cpm/ml, collagène IV 850.000 cpm/mg.

On prépare des PSE à 10 mg/ml dans PB 7,4. A 1 ml de cette solution, on ajoute 10, 20, 50, 100 et 200 µl de la solution de laminine ou de collagène IV. On incube 15 min. à 37 ° C. On ajoute qsp pour 2 ml (volume final) de PB 7,4 et 0,4 ml de collagène 1 + III. On homogénéise au VORTEX^{R} (5 sec) et conserve sans agitation 4 heures à 37 ° C. La centrifugation à 5500 t/min. pendant 40 min. permet de séparer culots et surnageants.

On constate que le gel résultant est d'autant plus compact que la quantité de laminine ou de collagène IV, est élevée. Des lavages répétés ou longs, ne libèrent pas la radio-activité liée aux culots, ce qui démontre l'affinité importante avec ces 2 protéines.

### EXEMPLE 6 : ETUDE DES PROPRIETES D'ADHE-SIVITE DE GEL PSE-COLLAGENE SUR DU DA- _{CRON}R

Un échantillon de prothèse en Dacron^{R -} (cylindre de 1 cm de longueur, 0,6 cm de diamètre) est mis à incuber dans du tampon PB de pH 7,4 pendant 4 heures. Dans le même temps, on prépare le gel : PSE-collagène en opérant comme suit : A 1 ml de PSE-1¹²⁵ (10 mg/ml), on ajoute 0,6 ml de TPS de pH 7,4 et 0,4 ml de collagène type III (10 mg/ml), en prenant bien soin d'homogénéiser entre chaque addition. On abandonne 4 heures à 37 ° C. Puis, on centrifuge 10 min. à 5000 t/min. et on lave 2 fois le culot par 2 ml d'eau permutée (pH 6). Four la longueur de prothèse utilisée, on a recours à 2 fois cette quantité de gel.

On applique alors le gel à la spatule à l'intérieur du matériel en Dacron^{R}, en l'étirant, puis on laisse sécher à l'air durant environ 14 heures. On obtient une prothèse un peu moins souple mais qui a retenu du matériel radio-actif.

Cette prothèse est alors lavée à l'eau (2 ml) sans agitation trop brusque pendant 5 heures, l'eau étant renouvelée après chaque mesure. A la 6e heure, on plonge la prothèse dans un bécher contenant 100 ml d'eau sous assez vive agitation magnétique. Au cours de tous ces lavages, la radio-activité restant sur la prothèse est évaluée jusqu'à la 23e heure.

Les résultats obtenus montrent que le gel PSE- collagène apparaît assez bien retenu sur le Dacron^{R} alors qu'il n'a pas été fortement fixé. Cette expérimentation confirme également, outre l'adhésivité, le caractère d'insolubilité du matériau dans l'eau.

### EXEMPLE 7 : PREPARATION D'UN MATERIAU COMPOSITE A BASE D'HYDROXYAPATITE, DE PEPTIDES SOLUBILISES D'ELASTINE ET DE COLLAGENE

On fait réagir tout d'abord de l'hydroxyapatite (ci-après hA) avec PSE-1¹²⁵ à 37 ° C, pH 7,4. On obtient un produit hA-PSE-l¹²⁵ ayant fixé 25 à 30% de la radioactivité.

On fait ensuite réagir ce produit avec du collagène de type I + III, à 37 ° C et pH 7,4.

Le produit résultant est un matériau composite du type hA-PSE-collagène, se présentant sous forme d'un gel autour de hA, qui reste solidement fixé après plusieurs lavages.

On utilise avantageusement PSE à raison de 20 mg/ml. Les sites d'association offerts par hA sont saturés avec 2 x 1 ml au bout de 15 min. d'incubation à 37 ° C.

La réaction de hA-PSE avec le collagène est réalisée par exemple avec 40 mg de hA-PSE, 1,2 ml de tampon à pH 7,4 et 0,8 ml (8 mg) de collagène 1 + III.

## Revendications (Revendications pour l'(les) Etat(s) contractant(s) suivant(s) : suivants : AT, BE, CH, DE, DK, FR, GB, IT, LI, LU, NL, SE)

1. Compositions de matériau protéique, caractérisées en ce qu'elles sont essentiellement telles qu'obtenues par réaction d'une solution aqueuse concentrée de collagène de type 1 et/ou III et d'élastine ou de peptides d'élastine appelés peptides solubilisés d'élastine, de poids moléculaire (PM) supérieur à environ 10.000, solubles dans l'eau, tels qu'obtenus par solubilisation de l'élastine.

2. Compositions selon la revendication 1, caractérisées en ce qu'elles sont essentiellement telles qu'obtenues par réaction de collagène de type 1 et de type III.

3. Compositions selon la revendication 2, caractérisées en ce que la proportion de collagène de type III est supérieure à 50 % en poids, en particulier d'au moins 70 % en poids, par rapport au poids sec.

4. Compositions selon la revendication 1, caractérisées en ce qu'elles sont essentiellement telles qu'obtenues par réaction de peptides solubilisés d'élastine et de collagène de type III.

5. Compositions selon l'une quelconque des revendications précédentes, caractérisées en ce que le rapport élastine ou peptides solubilisés d'élastine/collagène (p/p, par rapport au poids sec) est de 10 à 1 environ, de préférence de l'ordre de 2,5.

6. Compositions selon l'une quelconque des revendications précédentes, caractérisées en ce qu'elles sont essentiellement telles qu'obtenues par réaction en outre avec un matériau de substitution de l'os.

7. Compositions selon la revendication 6, caractérisées en ce que ledit matériau est biocompatible et doté de propriétés d'ostéoconduction et d'ostéoinduction.

8. Compositions selon la revendication 6 ou 7, caractérisées en ce que ledit matériau est à base de Ca^{++.}

9. Compositions selon l'une des revendications 1 à 8, caractérisées en ce que ledit biomatériau est à base de phosphate tricalcique ou d'apatites, en particulier d'hydroxyapatite.

10. Compositions selon la revendication 9, caractérisées en ce qu'elles sont telles qu'obtenues par réaction d'apatites, en particulier d'hydroxyapatite, avec des peptides solubilisés d'élastine et du collagène.

11. Compositions selon l'une des revendications 1 à 10, caractérisées en ce qu'elles comportent des additifs permettant d'améliorer leur qualité de gel et/ou de leur conférer des propriétés spécifiques, tels que notamment la fibronectine, la laminine, le collagène IV, des agents thérapeutiques et/ou antibactériens.

12. Procédé de préparation de compositions selon l'une quelconque des revendications 1 à 11, caractérisé en ce qu'il comprend :
- la mise en contact, avec une solution aqueuse concentrée de collagène de type 1 et/ou III, dans des conditions permettant la formation d'un gel, d'élastine ou de peptides solubilisés d'élastine de PM au moins égal à environ 10.000, ces peptides étant solubles dans un mélange 50-50 d'alcool tertiobutylique-potasse 1 M dans de l'eau,
- la récupération du gel formé et son traitement ultérieur, si souhaité.

13. Procédé selon la revendication 12, caractérisé en ce que l'étape de mise en contact est réalisée dans des conditions physiologiques, à savoir à 37 _{°} C environ, à un pH de l'ordre de 7 à 7,5.

14. Procédé selon la revendication 12 ou 13, caractérisé en ce qu'on utilise un tampon de pH de 7 à 7,5 environ, de préférence de 7,4, renfermant des cations Na⁺, Ca⁺⁺ et/ou Mg⁺⁺, des anions PO₄= et/ou CI-, en particulier le tampon PB de composition 1mM PO₄=, 150 mM NaCI, 2mM Ca⁺⁺, 1 mM Mg⁺⁺.

15. Procédé selon l'une quelconque des revendications 12 à 14, caractérisé en ce qu'on ajoute aux peptides solubilisés d'élastine ou à l'élastine, successivement, des additifs permettant d'améliorer les qualités du gel et de lui conférer des propriétés spécifiques, puis le collagène, le ou les additifs ajoutés étant choisis, notamment, parmi la fibronectine, la laminine, le collagène IV, des agents thérapeutiques et/ou antibactériens.

16. Procédé selon l'une quelconque des revendications 11 à 15, caractérisé en ce que pour obtenir les compositions selon l'une des revendications 6 à 10, on fait réagir le matériau de substitution de l'os avec des peptides solubilisés d'élastine, puis avec du collagène de type 1 et/ou III.

17. Compositions selon l'une des revendications 1 à 11, caractérisées en ce qu'elles sont mises en forme pour être utilisées comme membranes biologiques artificielles, par exemple sous forme de filaments, tubulures ou bandes.

18. Application des compositions selon l'une des revendications 1 à 11 et 17 pour la préparation de membranes biologiques artificielles, et notamment en tant que tissus conjonctifs artificiels ou comme pièces d'obturation, de soutien et de renforcement.

19. Utilisation de membranes biologiques artificielles selon la revendication 12, comme matériaux supports pour des cultures cellulaires, notamment de cultures de cellules musculaires lisses, de fibroblastes, de kératinocytes, de cellules épithéliales et de cellules endothéliales, ou encore pour la culture d'ostéoblastes en particulier avec les compositions des revendications 6 à 10, ou comme socle dermique pour l'ensemencement de cellules épithéliales

20. Application selon la revendication 18 ou 19, caractérisée en ce que lesdites compositions comprennent de l'héparane-sulfate, des chon- droltine-sulfates ou du kératane-sulfate.

21. Application selon l'une des revendications 18 à 20, comprenant l'adhésion des compositions aux matériaux supports polymères utilisés comme trame dans la préparation de biomatériaux.

22. Kits renfermant les compositions de matériau selon l'une des revendications 1 à 11 et 17 avec les éléments nécessaires pour effectuer une réparation tissulaire ou une culture cellulaire.

23. Membrane biologique artificielle élaborée à partir d'une composition de matériau protéique selon l'une des revendications 1 à 11.

## Revendications (Revendications pour l'(les) Etat(s) contractant(s) suivant(s) : suivant : ES)

1. Procédé de préparation de compositions de matériau protéique, caractérisé en ce qu'il comprend :
- la mise en contact, avec une solution aqueuse concentrée de collagène de type 1 et/ou III, dans des conditions permettant la formation d'un gel, d'élastine ou de peptides d'élastine, appelés peptides solubilisés d'élastine, de poids moléculaire (PM) au moins égal à environ 10.000, tels qu'obtenus par solubilisation de l'élastine, ces peptides étant solubles dans un mélange 50-50 d'alcool tertiobutylique-potasse 1 M dans de l'eau,
- la récupération du gel formé et son traitement ultérieur, si souhaité.

2. Procédé selon la revendication 1, caractérisé en ce que l'étape de mise en contact est réalisée dans des conditions physiologiques, à savoir à 37 ° C environ, à un pH de l'ordre de 7 à 7,5.

3. Procédé selon la revendication 1 ou 2, caractérisé en ce qu'on utilise un tampon de pH de 7 à 7,5 environ, de préférence de 7,4, renfermant des cations Na⁺, Ca⁺⁺ et/ou Mg⁺⁺, des anions PO₄= et/ou CI-, en particulier le tampon PB de composition 1 mM P04=, 150 mM NaCI, 2mM Ca⁺⁺, 1 mM Mg⁺⁺.

4. Procédé selon l'une des revendications 1 à 3, caractérisé en ce qu'on utilise du collagène de type 1 et de type III.

5. Procédé selon la revendication 4, caractérisé en ce que la proportion de collagène de type III est supérieure à 50 % en poids, en particulier d'au moins 70 % en poids, par rapport au poids sec.

6. Procédé selon l'une des revendications 1 à 3, caractérisé en ce qu'on fait réagir des peptides solubilisés d'élastine et du collagène de type III.

7. Procédé selon l'une quelconque des revendications précédentes, caractérisé en ce que le rapport élastine ou peptides solubilisés d'élastine/ collagène (p/p, par rapport au poids sec) est de 10 à 1 environ, de préférence de l'ordre de 2,5.

8. Procédé selon l'une quelconque des revendications précédentes, caractérisé en ce qu'on ajoute aux peptides solubilisés d'élastine ou à l'élastine, successivement, des additifs permettant d'améliorer les qualités du gel et de lui conférer des propriétés spécifiques, puis le collagène, le ou les additifs ajoutés étant choisis, notamment, parmi la fibronectine, la laminine, le collagène IV, des agents thérapeutiques et/ou antibactériens.

9. Procédé selon l'une quelconque des revendications précédentes, caractérisé en ce qu'on fait réagir un matériau de substitution de l'os avec l'élastine ou les peptides solubilisés d'élastine, puis avec du collagène de type 1 et/ou III.

10. Procédé selon la revendication 9, caractérisé en ce que ledit matériau est biocompatible et doté de propriétés d'ostéoconduction et d'os- téoinduction.

11. Procédé selon la revendication 9 ou 10, caractérisé en ce que ledit matériau est à base de Ca^{++.}

12. Procédé selon l'une des revendications 9 à 11, caractérisé en ce que ledit matériau est à base de phosphate tricalcique ou d'apatites, en particulier d'hydroxyapatite.

13. Procédé selon l'une quelconque des revendications précédentes, caractérisé en ce que les compositions obtenues sont mises en forme pour être utilisées comme membranes biologiques artificielles, par exemple sous forme de filaments, tubulures ou bandes.

14. Application des compositions de matériau protéique telles qu'obtenues selon l'une quelconque des revendications 1 à 13, pour la préparation de membranes biologiques artificielles, et notamment en tant que tissus conjonctifs artificiels ou comme pièces d'obturation, de soutien et de renforcement.

15. Utilisation de membranes biologiques artificielles selon la revendication 13, comme matériaux supports pour des cultures cellulaires, notamment des cultures de cellules musculaires lisses, de fibroblastes, de kératinocytes, de cellules épithéliales et de cellules endothéliales, ou encore pour la culture d'ostéoblastes en particulier en utilisant les compositions telles qu'obtenues selon l'une des revendications 9 à 12, ou comme socle dermique pour l'ensemencement de cellules épithéliales.

16. Application selon la revendication 14 ou 15, caractérisée en ce que lesdites compositions comprennent de l'héparane-sulfate, des chon- droltine-sulfates ou du kératane-sulfate.

17. Application selon l'une des revendications 14 à 16, comprenant l'adhésion des compositions aux matériaux supports polymères utilisés comme trame dans la préparation de biomatériaux.

18. Kits renfermant les compositions de matériau protéique telles qu'obtenues selon l'une des revendications 1 à 12 avec les éléments nécessaires pour effectuer une réparation tissulaire ou une culture cellulaire.

## Claims (Claims for the following Contracting State(s) : AT, BE, CH, DE, DK, FR, GB, IT, LI, LU, NL, SE)

1. Compositions of protein material, characterized in that they are essentially as obtained by the reaction of a concentrated aqueous solution of type I and/or III collagen and elastin or elastin peptides, so-called solubilized elastin peptides, having a molecular weight (MW) greater than approximately 10000, as obtained by the solubilization of elastin.

2. Compositions according to claim 1, characterized in that they are essentially as obtained by the reaction of type I and type III collagen.

3. Compositions according to claim 2, characterized in that the proportion of type III collagen is greater than 50% by weight, more particularly at least 70% by weight, in relation to the dry weight.

4. Compositions according to claim 1, characterized in that they are essentially as obtained by the reaction of solubilized elastin peptides and type III collagen.

5. Compositions according to any of the preceding claims, characterized in that the elastin or solubilized elastin peptides/collagen ratio (w/w in relation to the dry weight) is approximately 10 to 1, preferably of the order of 2.5.

6. Compositions according to any of the preceding claims, characterized in that they are essentially as obtained by also reacting with a bone substitute material.

7. Compositions according to claim 6, wherein said material is biocompatible and possesses properties of osteoconduction and osteoinduction.

8. Compositions according to claim 6 or 7, wherein said material is based on Ca + +.

9. Compositions according to anyone of claims 1 to 8, wherein said biomaterial is based on tricalcic phosphate or apatites, particularly hydroxyapatite.

10. Compositions according to claim 9, characterized in that they are such as obtained by reaching apatites, particularly hydroxyapatite, with solubilized elastin peptides and collagen.

11. Compositions according to anyone of claims 1 to 10, comprising additives enabling the qualities of the gel to be improved and/or the gel to be given specific properties, such as particularly fibronectin, laminin, collagen IV, therapeutic and/or antibacterial agents.

12. A process for the preparation of compositions according to anyone of claims 1 to 11, comprising :
- contacting, with a concentrated aqueous solution of type I and/or III collagen, in conditions allowing the formation of a gel, elastin or solubilized elastin peptides having a MW at least equal to approximately 10000, said peptides being soluble in a 50-50 mixture of tertiobutylic alcohol-1 M potassium hydroxide in water,
- the recovery of the gel formed and its subsequent treatment, if desired.

13. A process according to claim 12, wherein the contacting stage is performed in physiological conditions - i.e., at approximately 37 ° C with a pH of the order of 7 to 7.5.

14. A process according to Claim 12 or 13, wherein use is made of a buffer of pH approximately 7 to 7.5, preferably 7.4, containing Na⁺, Ca⁺⁺ and/or Mg⁺⁺ cations, PO₄= and/or CI- anions, more particularly the PB buffer of composition 1mM PO₄-, 150 mM NaCI, 2mM Ca⁺⁺, 1mM Mg⁺⁺.

15. A process according to any one of claims 12 to 14, characterized in that there are added successively to elastin or the solubilized elastin peptides, additives enabling the qualities of the gel to be improved and the gel to be given specific properties, and thereafter the collagen, the or each additive added being selected more particularly from fibronectin, laminin, IV collagen, therapeutic and/or antibacterial agents.

16. A process according to anyone of claims 11 to 15, wherein to obtain the compositions according to anyone of claims 6 to 10, the bone substitution material is reacted with solubilized elastin peptides, then type I and/or III collagen.

17. Compositions according to anyone of claims 1 to 11, characterized in that they are shaped to be used as artificial biological membranes, for example into the form of filaments, tubes or strips.

18. Application of the composition according to anyone of claims 1 to 11 and 17 for the preparation of artificial biological membranes, and particularly as artificial connective tissues or closure support and reinforcing members.

19. Use of artificial biological membranes according to claim 12, as support materials for cell cultures, particularly cultures, particularly cultures of smooth muscle cells, fibroblasts, keratinocytes, epithelial cells or endothelial cells, or for osteoblast cultures, particularly using the compositions such as obtained according to claims 6 to 10, or epidermis interface for seeding epithalial cells.

20. Application according to claim 18 or 19, wherein said composition comprise heparan sulphate, chondroitin sulphates or keratane sulphate.

21. Application according to anyone of claims 18 to 20, comprising adhering said compositions to polymeric supporting materials used as webs in the preparation of biomaterials.

22. Kits containing the proteinaceous compositions such as obtained according to anyone of claims 1 to 11 and 17 with the elements necessary to effect a tissue repair or a cell culture.

23. Artificial biological membrane elaborated from a protein material composition according to anyone of claims 1 to 11.

## Claims (Claims for the following Contracting State(s) : ES)

1. A process for the preparation of proteinaceous compositions comprising :
- contacting, with a concentrated aqueous solution of type I and/or III collagen, under conditions allowing the formation of a gel, elastin or elastin peptides, so-called solubilized elastin peptides, having a molecular weight (MW) of approximately 10,000, such as obtained by the solubilization of elastin, said peptides being soluble in a 50/50 mixture of tertiobutylic alcohol - 1 M potassium hydroxide in water; and
- recovering the gel formed thereby, and subsequently, treating the same, if desired.

2. A process according to claim 1, wherein the contacting step is carried out under physiological conditions, i. e. at about 37 ° C, with a pH of the order of 7 to 7.5.

3. A process according to claim 1 or 2, wherein a buffer with a pH of about 7.0 to 7.5, preferably of 7.4 is used, containing Na⁺, Ca⁺⁺ and/or Mg⁺⁺ cations, PO₄= and/or CI- anions, particularly the PB buffer of composition 1 mM PO₄=,150 mM NaCI, 2mM Ca⁺⁺, 1mM Mg^{++.}

4. A process according to anyone of claims 1 to 3, wherein type I and type III collagen is used.

5. A process according to claim 4, wherein the proportion of type III collagen is greater than 50% (w/w), particularly of at least 70% (w/w) by dry weight.

6. A process according to anyone of claims 1 to 3, wherein elastin solubilized peptides are reacted with the type III collagen.

7. A process according to anyone of the preceding claims, wherein the ratio of elastin or solubilized elastin peptides/collagen (w/w, by dry weight) is of about 10 to 1, preferably of about 2.5.

8. A process according to anyone of the preceding claims, wherein additives enabling the qualities of the gel to be improved and the gel to be given specific properties, are successively added to the solubilized elastin peptides or to elastin, followed by collagen, the additives being particularly selected among fibronectin, laminin, collagen IV, therapeutic and/or antibacterial agents.

9. A process according to anyone of the preceding claims, wherein a bone substitute material is reacted with elastin or solubilized elastin peptides, then with type I and/or III collagen.

10. A process according to claim 9, wherein said material is biocompatible and possesses properties of osteoconduction and osteoinduction.

11. A process according to claim 9 or 10, wherein said material is based on Ca⁺⁺.

12. A process according to anyone of claims 9 to 11, wherein said material is based on tricalcic phosphate or apatites, particularly hydroxyapatite.

13. A process according to anyone of the preceding claim, wherein the obtained compositions are shaped to be used as artificial biological membranes, for example into the form of filaments, tubes or strips.

14. Use of proteinaceous compositions such as obtained according to anyone of claims 1 to 13 for the preparation of artificial biological membranes, and particularly as artificial connective tissues or closure, support and reinforcing members.

15. Use of artificial biological membranes according to claim 13, as support materials for cell cultures, particularly cultures, particularly cultures of smooth muscle cells, fibroblasts, keratinocytes, epithelial cells or endothelial cells, or for osteoblast cultures, particularly using the compositions such as obtained according to anyone claims 9 to 12, or as epidermis interface for seeding epithalial cells.

16. Application according to claim 14 or 15, wherein said compositions comprise heparan sulphate, chondroitin sulphates or keratane sulphate.

17. Application according to anyone of claims 14 to 16, comprising adhering said compositions to polymeric supporting materials used as webs in the preparation of biomaterials.

18. Kits containing the proteinaceous compositions such as obtained according to anyone of claims 1 to 12 with the elements necessary to effect a tissue repair or a cell culture.

## Patentansprüche (Patentansprüche für folgende(n) Vertragsstaat(en) : AT, BE, CH, DE, DK, FR, GB, IT, LI, LU, NL, SE)

1. Proteinmaterial-Zusammensetzungen, dadurch gekennzeichnet, daß es sich im wesentlichen um jene handelt, welche erhalten werden durch Umsetzung einer konzentrierten wäßrigen Lösung von Kollagen des Typs I und/oder III mit Elastin oder Elastinpeptiden, welche als solubilisierte Elastinpeptide bezeichnet werden, mit Molekulargewichten (MG) über etwa 10.000, welche in Wasser löslich sind, wie jene, die durch Solubilisierung von Elastin erhalten werden.

2. Zusammensetzungen nach Anspruch 1, dadurch gekennzeichnet, daß es sich dabei im wesentlichen um jene handelt, welche durch Umsetzung von Kollagen des Typs I und des Typs III erhalten werden.

3. Zusammensetzungen nach Anspruch 2, dadurch gekennzeichnet, daß der Anteil an Kollagen des Typs III über 50 Gew.-% und insbesondere mindestens 70 Gew.-%, bezogen auf das Trockengewicht, beträgt.

4. Zusammensetzungen nach Anspruch 1, dadurch gekennzeichnet, daß es sich dabei im wesentlichen um jene handelt, welche durch Umsetzung von solubilisierten Elastinpeptiden mit Kollagen vom Typ III erhalten werden.

5. Zusammensetzungen nach einem der vorstehenden Ansprüche, dadurch gekennzeichnet, daß das Verhältnis von Elastin oder den solubilisierten Elastinpeptiden zu Kollagen (Gewicht/Gewicht, bezogen auf das Trockengewicht) ungefähr 10 zu 1 beträgt, vorzugsweise in der Größenordnung von 2,5 liegt.

6. Zusammensetzungen nach einem der vorstehenden Ansprüche, dadurch gekennzeichnet, daß es sich dabei im wesentlichen um jene handelt, welche durch die zusätzliche Umsetzung mit einem Knochenersatzmaterial erhalten werden.

7. Zusammensetzungen nach Anspruch 6, dadurch gekennzeichnet, daß das genannte Material biologisch verträglich ist und die Eigenschaften der Knochenleitung und der Knocheninduktion besitzt.

8. Zusammensetzungen nach Anspruch 6 oder 7, dadurch gekennzeichnet, daß das genannte Material auf Ca⁺⁺ basiert.

9. Zusammensetzungen nach einem der Ansprüche 1 bis 8, dadurch gekennzeichnet, daß das genannte Biomaterial auf Tricalciumphosphat oder Apatiten, insbesondere auf Hydroxyapatit, basiert.

10. Zusammensetzungen nach Anspruch 9, dadurch gekennzeichnet, daß es sich dabei um jene handelt, welche durch Umsetzung von Apatiten, insbesondere von Hydroxyapatit, mit den solubilisierten Elastinpeptiden und Kollagen erhalten werden.

11. Zusammensetzungen nach einem der Ansprüche 1 bis 10, dadurch gekennzeichnet, daß sie Zusatzstoffe umfassen, welche es ermöglichen, deren Gelqualität zu verbessern und/oder diesen spezielle Eigenschaften zu verleihen, wie insbesondere Fibronectin, Laminin, Kollagen IV, therapeutische und/oder antibakterielle Mittel.

12. Verfahren zur Herstellung der Zusammensetzungen nach einem der Ansprüche 1 bis 11, dadurch gekennzeichnet, daß es:
- das Inkontaktbringen von Elastin oder solubilisierten Elastinpeptiden mit einem Molekulargewicht von mindestens etwa 10.000, welche Peptide in einem Gemisch von tert.Butylalkohol und 1 M Kaliumhydroxid in Wasser im Verhältnis 50:50 löslich sind, mit einer wäßrigen konzentrierten Lösung von Kollagen des Typs I und/oder 111, unter Bedingungen, welche die Bildung eines Geles ermöglichen,
- die Gewinnung des gebildeten Geles und gewünschtenfalls seine weitere Behandlung umfaßt.

13. Verfahren nach Anspruch 12, dadurch gekennzeichnet, daß der Schritt des Inkontaktbringens unter physiologischen Bedingungen, nämlich bei etwa 37 °C, bei einem pH-Wert in der Größenordnung von 7 bis 7,5 durchgeführt wird.

14. Verfahren nach Anspruch 12 oder 13, dadurch gekennzeichnet, daß man einen Puffer mit einem pH-Wert von etwa 7 bis 7,5, vorzugsweise 7,4, welcher Na⁺⁻, Ca⁺⁺- und/oder Mg⁺⁺-Kationen, PO₄=- und/oder CI--Anionen, enthält, insbesondere den Puffer PB der Zusammensetzung 1mM PO₄=, 150 mM NaCI, 2mM Ca++, 1 mM Mg⁺⁺, verwendet.

15. Verfahren nach einem der Ansprüche 12 bis 14, dadurch gekennzeichnet, daß man zu den solubilisierten Elastinpeptiden oder dem Elastin nacheinander Zusatzstoffe, welche es ermöglichen, die Gelqualitäten zu verbessern und diesen spezielle Eigenschaften zu verleihen, danach das Kollagen zusetzt, wobei der oder die zugesetzten Zusatzstoffe insbesondere unter Fibronectin, Laminin, Kollagen IV, therapeutischen und/oder antibakteriellen Mitteln ausgewählt sind.

16. Verfahren nach einem der Ansprüche 11 bis 15, dadurch gekennzeichnet, daß man das Knochenersatzmaterial mit solubilisierten Elastinpeptiden und dann mit Kollagen des Typs I und/oder III umsetzt, um die Zusammensetzungen nach einem der Ansprüche 6 bis 10 zu erhalten.

17. Zusammensetzungen nach einem der Ansprüche 1 bis 11, dadurch gekennzeichnet, daß man diese für die Verwendung als künstliche biologische Membranen in eine Form, insbesondere in die Form von Fasern, Röhren oder Bändern, überführt.

18. Verwendung der Zusammensetzungen nach einem der Ansprüche 1 bis 11 und 17 zur Herstellung von künstlichen biologischen Membranen und insbesondere als künstliche Bindehautgewebe oder als Verschluß-, Festigungs-und Verstärkungsstücke.

19. Verwendung der künstlichen biologischen Membranen nach Anspruch 12, als Trägermaterialien für Zellkulturen, insbesondere für Kulturen von glatten Muskelzellen, Fibroblasten, Keratinocyten, Epithelzellen und Endothelzellen, oder auch für die Osteoblastenkultur, insbesondere mit den Zusammensetzungen der Ansprüche 6 bis 10, oder als Dermalsockel für die Beimpfung mit Epithelzellen.

20. Verwendung nach Anspruch 18 oder 19, dadurch gekennzeichnet, daß die genannten Zusammensetzungen Heparansulfat, Chondroitinsulfate oder Keratansulfat umfassen.

21. Verwendung nach einem der Ansprüche 18 bis 20, umfassend die Haftung der Zusammensetzungen auf polymeren Trägermaterialien, welche als Grundlage für die Herstellung von Biomaterialien verwendet werden.

22. Kits, enthaltend die Material-Zusammensetzungen nach einem der Ansprüche 1 bis 11 und 17 und die für das Erreichen einer Wiederherstellung von Gewebe und/oder einer Zellkultur erforderlichen Bestandteile.

23. Künstliche biologische Membran, hergestellt aus einer Proteinmaterial-Zusammensetzung nach einem der Ansprüche 1 bis 11.

## Patentansprüche (Patentansprüche für folgende(n) Vertragsstaat(en) : ES)

1. Verfahren zur Herstellung von Proteinmaterial-Zusammensetzungen, dadurch gekennzeichnet, daß es:
- das Inkontaktbringen von Elastin oder Elastinpeptiden, welche als solubilisierte Elastinpeptide bezeichnet werden, mit Molekulargewichten (MG) von mindestens etwa 10.000, wie jene, die durch Solubilisierung von Elastin erhalten werden, welche Peptide in einem Gemisch von tert.Butylalkohol und 1 M Kaliumhydroxid in Wasser im Verhältnis 50:50 löslich sind, mit einer wäßrigen konzentrierten Lösung von Kollagen des Typs I und/oder 111, unter Bedingungen, welche die Bildung eines Geles ermöglichen,
- die Gewinnung des gebildeten Geles und gewünschtenfalls seine weitere Behandlung umfaßt.

2. Verfahren nach Anspruch 1, dadurch gekennzeichnet, daß der Schritt des Inkontaktbringens unter physiologischen Bedingungen, nämlich bei etwa 37 ° C, bei einem pH-Wert in der Größenordnung von 7 bis 7,5 durchgeführt wird.

3. Verfahren nach Anspruch 1 oder 2, dadurch gekennzeichnet, daß man einen Puffer mit einem pH-Wert von etwa 7 bis 7,5, vorzugsweise 7,4, welcher Na⁺⁻, Ca⁺⁺- und/oder Mg⁺⁺-Kationen, PO₄= - und/oder CI--Anionen, enthält, insbesondere den Puffer PB der Zusammensetzung 1mM PO₄=, 150 mM NaCI, 2mM Ca++, 1 mM Mg++, verwendet.

4. Verfahren nach einem der Ansprüche 1 bis 3, dadurch gekennzeichnet, daß man Kollagen des Typs I und des Typs III verwendet.

5. Verfahren nach Anspruch 4, dadurch gekennzeichnet, daß der Anteil an Kollagen des Typs III über 50 Gew.-% und insbesondere mindestens 70 Gew.-%, bezogen auf das Trockengewicht, beträgt.

6. Verfahren nach einem der Ansprüche 1 bis 3, dadurch gekennzeichnet, daß man solubilisierte Elastinpeptide mit Kollagen vom Typ III umsetzt.

7. Verfahren nach einem der vorstehenden Ansprüche, dadurch gekennzeichnet, daß das Verhältnis von Elastin oder den solubilisierten Elastinpeptiden zu Kollagen (Gewicht/Gewicht, bezogen auf das Trockengewicht) ungefähr 10 zu 1 beträgt, vorzugsweise in der Größenordnung von 2,5 liegt.

8. Verfahren nach einem der vorstehenden Ansprüche, dadurch gekennzeichnet, daß man zu den solubilisierten Elastinpeptiden oder dem Elastin nacheinander Zusatzstoffe, welche es ermöglichen, die Gelqualitäten zu verbessern und diesen spezielle Eigenschaften zu verleihen, danach das Kollagen zusetzt, wobei der oder die zugesetzten Zusatzstoffe insbesondere unter Fibronectin, Laminin, Kollagen IV, therapeutischen und/oder antibakteriellen Mitteln ausgewählt sind.

9. Verfahren nach einem der vorstehenden Ansprüche, dadurch gekennzeichnet, daß man das Knochenersatzmaterial mit Elastin oder den solubilisierten Elastinpeptiden und dann mit Kollagen des Typs I und/oder 111 umsetzt.

10. Verfahren nach Anspruch 9, dadurch gekennzeichnet, daß das genannte Material biologisch verträglich ist und die Eigenschaften der Knochenleitung und der Knocheninduktion besitzt.

11. Verfahren nach Anspruch 9 oder 10, dadurch gekennzeichnet, daß das genannte Material auf Ca++ basiert.

12. Verfahren nach einem der Ansprüche 9 bis 11, dadurch gekennzeichnet, daß das genannte Material auf Tricalciumphosphat oder Apatiten, insbesondere auf Hydroxyapatit, basiert.

13. Verfahren nach einem der vorstehenden Ansprüche, dadurch gekennzeichnet, daß die erhaltenen Zusammensetzungen für die Verwendung als künstliche biologische Membranen in eine Form, insbesondere in die Form von Fasern, Röhren oder Bändern, übergeführt werden.

14. Verwendung der nach einem der Ansprüche 1 bis 13 erhaltenen Proteinmaterial-Zusammensetzungen zur Herstellung von künstlichen biologischen Membranen und insbesondere als künstliche Bindehautgewebe oder als Verschluß-, Festigungs- und Verstärkungsstücke.

15. Verwendung der künstlichen biologischen Membranen nach Anspruch 13, als Trägermaterialien für Zellkulturen, insbesondere für Kulturen von glatten Muskelzellen, Fibroblasten, Keratinocyten, Epithelzellen und Endothelzellen, oder auch für die Osteoblastenkultur, wobei man insbesondere die nach einem der Ansprüche 9 bis 12 erhaltenen Zusammensetzungen verwendet, oder als Dermalsockel für die Beimpfung mit Epithelzellen.

16. Verwendung nach Anspruch 14 oder 15, dadurch gekennzeichnet, daß die genannten Zusammensetzungen Heparansulfat, Chondroitinsulfate oder Keratansulfat umfassen.

17. Verwendung nach einem der Ansprüche 14 bis 16, umfassend die Haftung der Zusammensetzungen auf polymeren Trägermaterialien, welche als Grundlage für die Herstellung von Biomaterialien verwendet werden.

18. Kits, enthaltend die nach einem der Ansprüche 1 bis 12 erhaltenen Proteinmaterial-Zusammensetzungen und die für das Erreichen einer Wiederherstellung von Gewebe und/oder einer Zellkultur erforderlichen Bestandteile.
